(19) Europäisches Patentamt
European Patent Office
Office européen des brevets

(11) Veröffentlichungsnummer: **0 056 488**
**B1**

(12) **EUROPÄISCHE PATENTSCHRIFT**

(45) Veröffentlichungstag der Patentschrift: **07.03.84**

(51) Int. Cl.[3]: **C 07 C 31/08**, C 07 C 29/136, C 07 C 29/80, C 07 C 29/92, C 07 C 69/14, C 07 C 67/36

(21) Anmeldenummer: **81110726.7**

(22) Anmeldetag: **23.12.81**

(54) **Verfahren zur kontinuierlichen Herstellung von Ethanol.**

(30) Priorität: **21.01.81 DE 3101750**

(43) Veröffentlichungstag der Anmeldung: **28.07.82 Patentblatt 82/30**

(45) Bekanntmachung des Hinweises auf die Patenterteilung: **07.03.84 Patentblatt 84/10**

(84) Benannte Vertragsstaaten: **BE DE FR GB IT NL**

(56) Entgegenhaltungen:
**DE - A - 2 726 978**
**FR - A - 1 006 012**
**GB - A - 1 277 242**
**US - A - 2 782 243**

(73) Patentinhaber: **BASF Aktiengesellschaft, Carl-Bosch-Strasse 38, D-6700 Ludwigshafen (DE)**

(72) Erfinder: **Kummer, Rudolf, Dr., Kreuzstrasse 6, D-6710 Frankenthal (DE)**
Erfinder: **Taglieber, Volker, Dr., Franz-Liszt-Strasse 15, D-6904 Eppelheim (DE)**
Erfinder: **Schneider, Heinz-Walter, Dr., Bruesseler Ring 43, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Verfahren zur kontinuierlichen Herstellung von Ethanol

Die vorliegende Erfindung betrifft ein neues Verfahren zur kontinuierlichen Gewinnung von Ethanol.

Es ist allgemein bekannt, dass man Methanol nach verschiedenen Methoden der Carbonylierung in Ethanol überführen kann, beispielsweise durch ein- oder zweistufige Homologisierung gemäss den folgenden Reaktionsgleichungen:

$$CH_3OH + CO + 2H_2 \xrightarrow{Kat.} CH_3CH_2OH + H_2O$$

bzw.

$$3\,CH_3OH + CO + H_2 \xrightarrow{Kat.} CH_3CH(OCH_3)_2 + 2\,H_2O$$

$$\xrightarrow{H_2} CH_3CH_2OH + 2\,CH_3OH$$

Wie aus neueren Untersuchungen hervorgeht (Ind. Eng. Chem. Prod. Res. Dev., Band 17/3 (978), S. 231f.; DE-OS 27 26 978), sind hierbei jedoch nur Ethanol-Ausbeuten von 50 bis 60% zu erzielen.

Eine weitere Möglichkeit, Ethanol aus Methanol herzustellen, besteht in der Carbonylierung von Methanol zu Methylacetat und der anschliessenden Hydrierung des Methylacetates (s. z.B. FR-A-1 006 012):

$$2\,CH_3OH + CO \xrightarrow{Kat.} CH_3CO\text{-}OCH_3 + H_2O$$

$$\xrightarrow{H_2} CH_3CH_2OH + CH_3OH$$

Da man bei der Carbonylierung von Methanol jedoch kein reines Methylacetat sondern ein Gemisch aus Methylacetat, Essigsäure, Methanol, Dimethylether, Wasser, meistens auch jodorganischen Verbindungen und von sonstigen Nebenprodukten erhält, fand diese Methode der Ethanolgewinnung wegen der grossen verfahrenstechnischen Probleme bisher keinen Eingang in die Technik.

Entgegen dem hierdurch begründeten Vorurteil war es die Aufgabe der Erfindung, die Carbonylierung von Methanol für die Ethanolgewinnung technisch und wirtschaftlich nutzbar zu machen.

Dementsprechend wurde ein kontinuierliches Verfahren zur Herstellung von Ethanol auf dem Wege über die Carbonylierung von Methanol gefunden, welches dadurch gekennzeichnet ist, dass man

a) Methanol in Gegenwart eines Carbonylkomplexes eines Metalles der VIII. Gruppe des Periodensystems sowie einer Halogenverbindung als Aktivator in an sich bekannter Weise in einem Reaktor R carbonyliert,

b) den im wesentlichen aus Methylacetat, Methanol, Essigsäure, Wasser sowie geringen Mengen Dimethylether, Methyljodid und, falls kein fest angeordneter Katalysator verwendet wird, dem Katalysator bestehenden Reaktoraustrag in einer Destillationskolonne D1 in eine Kopffraktion aus Methylacetat, Methanol, Dimethylether und einer halogenorganischen Verbindung einerseits und eine Sumpffraktion aus Wasser, geringen Mengen Essigsäure und dem Katalysator andererseits trennt, wobei man die Verweilzeit so einstellt, dass der Grossteil der Essigsäure mit dem vorhandenen Methanol zu Methylacetat reagiert,

c) Die Kopffraktion aus D1 in einer Destillationskolonne D2 in eine Kopffraktion aus geringen Mengen Methylacetat, Methanol, Dimethylether und der halogenorganischen Verbindung einerseits und eine Sumpffraktion aus Methylacetat und Methanol andererseits zerlegt und die Kopffraktion nach R zurückführt,

d) aus der Sumpffraktion von D1 in einer Destillationskolonne D3 den Grossteil des Wassers über Kopf abdestilliert und aus dem Kreislauf entfernt und die aus geringen Mengen Wasser, Essigsäure und dem Katalysator bestehende Sumpffraktion nach R zurückführt und

e) die Sumpffraktion von D2 in den Hydrierreaktor H in an sich bekannter Weise mit Wasserstoff zu einem Gemisch aus Methanol und Ethanol hydriert, welches

f) in einer Destillationskolonne D4 in Ethanol und Methanol zerlegt wird, wonach das Methanol nach R zurückgeführt wird.

Figur 1, die sich aufgrund der vorstehenden Erfindungsbeschreibung aus sich selbst heraus versteht, veranschaulicht das Verfahrensschema für den Fall, dass ein Jodid als Aktivator verwendet wird, wobei man als halogenorganische Verbindung hauptsächlich Methyljodid erhält.

Figur 2 zeigt eine apparative Variante dieses Verfahrens, nach welcher die Funktionen der Destillationskolonnen D1, D2 und D3 in einer einzigen Kolonne vereinigt werden, wobei die Methylacetat/Methanol-Fraktion und die Wasser-Fraktion als Seitenabzüge entnommen werden.

Der Verfahrensschrit (a), die Carbonylierung von Methanol, ist in zahlreichen Varianten bekannt. Allgemein nimmt man ihn bei 50 bis 300°C und unter einem CO-Druck von 1 bis 800 bar in Gegenwart eines Carbonyl-Komplexes eines Metalles der VIII. Gruppe des Periodensystems als Katalysator sowie einer Halogenverbindung als Aktivator vor.

Als Zentralatome in den Katalysatoren kommen vor allem Cobalt, Nickel und besonders Rhodium in Betracht. Ausserdem ist es zweckmässig, nicht die reinen, relativ instabilen Carbonylkomplexe zu verwenden, sondern solche, in denen ein Teil der CO-Liganden zur Erhöhung der Stabilität der Komplexe durch andere Liganden ersetzt ist. Solche Liganden sind bekanntermassen vor allem organische Verbindungen des dreiwertigen

Phosphors wie $C_4$-$C_{12}$-Trialkylphosphine und -phosphite und Triarylphosphine und -phosphite. Besonders bevorzugt wird Triphenylphosphin.

Man kann die fertigen Komplexverbindungen, z.B. $Rh(CO)(PPh_3)_2Cl$ (Ph = Phenyl) einsetzen, jedoch ist es im allgemeinen wirtschaftlicher und verfahrenstechnisch einfacher, von den Salzen der entsprechenden Metalle sowie gegebenenfalls den Liganden auszugehen, weil sich die Komplexe unter den Reaktionsbedinungen in situ bilden.

Pro Kilogramm Methanol sollen im Reaktor zweckmässigerweise $10^{-4}$ bis 1 Grammatom des Katalysatormetalls zugegen sein, und die Menge der Liganden beträgt vorteilhaft 2 bis 100 mol pro Grammatom des Metalls. Der molare Überschuss der Liganden dient dazu, das wegen des grossen CO-Angebots zur Seite der reinen Carbonylkomplexe tendierende Gleichgewicht zugunsten der gemischten Komplexe zu verschieben.

Als Aktivatoren eignen sich bekanntermassen Halogenverbindungen wie Chloride, Bromide und vor allem Jodide. Man setzt diese Verbindungen z.B. als Salze wie LiJ oder NaJ oder in Form der Halogenwasserstoffsäuren ein. Im Laufe der Reaktion bilden sich hieraus organische Halogenide, darunter vor allem die Methylhalogenide, so dass es sich wegen der Bevorzugung des Jodes in der Praxis meistens um Methyljodid handelt. Die Menge dieser Aktivatoren beträgt vorzugsweise $10^{-2}$ bis $10^{-3}$ mol Halogen pro Grammatom des Katalysatormetalls.

Die Mitverwendung von Lösungsmitteln ist möglich, im allgemeinen aber nicht erforderlich. Als Lösungsmittel eignen sich Essigsäure, höhere Carbonsäuren, hochsiedende Carbonsäureester und Kohlenwasserstoffe, die über 100°C sieden.

Im allgemeinen empfiehlt es sich, den Methanolumsatz auf etwa 50 bis 80% zu begrenzen, da man bei höheren Umsätzen mit einer verstärkten Bildung von Nebenprodukten zu rechnen hat. Soweit diese Nebenprodukte gebildet werden, müssen sie in üblicher Weise aus dem Kreislauf entfernt werden, und zwar, da sie höher als die übrigen Komponenten sieden, aus dem Sumpfprodukt der Kolonne D3, wie in die Figuren der Übersichtlichkeit wegen nicht eingezeichnet wurde. Inwieweit der Vorteil des höheren Methanolumsatzes den Nachteil der erhöhten Nebenproduktbildung und -entfernung kompensiert, ist eine Frage üblicher wirtschaftlicher Optimierung, die von den Gegebenheiten des Einzelfalls abhängt.

Bezogen auf den Methanolumsatz (= 100%) erhält man bei der Carbonylierung etwa 10 bis 50% Methylacetat, 5 bis 40% Essigsäure, 5 bis 20% Dimethylether, 5 bis 10% Wasser neben geringen Mengen sonstiger Produkte, wie Ethanol, Propionsäure und Propionsäureester.

Im Rahmen des erfindungsgemässen Verfahrens ist es besonders zweckmässig, die Carbonylierung bei 150 bis 200°C und 5 bis 50 bar mittels eines Rh/Triphenylphosphinkatalysators und eines Jodides als Aktivator in der Flüssigphase vorzunehmen. Die Menge des Rhodiums beträgt hierbei $10^{-3}$ bis $10^{-1}$ Gew.%, bezogen auf das im Reaktor vorhandene Methanol, die Menge des Triphenylphosphins 5 bis 100 mol pro Grammatom Rhodium und die Menge des Jodides (eingesetzt als Jodwasserstoff oder Methyljodid) 10 bis 100 mol J pro Grammatom des Rhodiums. Der Methanolumsatz wird hierbei auf 70 bis 80% begrenzt, so dass als Reaktoraustrag ein Gemisch aus 35 bis 50 Gew.% Methylacetat, 5 bis 20 Gew.% Methanol, 5 bis 15 Gew.% Essigsäure, 5 bis 20 Gew.% Wasser, 1 bis 15 Gew.% Dimethylether, bis zu 5 Gew.% sonstiger Produkte und dem Rest an Katalysatorbestandteilen erhält. Die Raum-Zeit-Ausbeute für die erwünschten Verfahrensprodukte Methylacetat und Essigsäure beträgt für die bevorzugte Ausführungsform des Carbonylierungsschrittes des erfindungsgemässen Verfahrens etwa 0,1 bis 0,6 kg/l/h.

Man kann die Carbonylierung aber auch in an sich bekannter Weise an einem fest angeordneten Katalysator in der Gasphase vornehmen. In diesem Falle enthält der Stoffkreislauf keine metallischen Katalysatorbestandteile.

Das nach Verfahrensschritt (a) erhaltene Reaktionsgemisch wird sodann gemäss Verfahrensschritt (b) der Destillationskolonne D1 zugeführt. Diese Kolonne hat die Funktion, die leicht siedenden Bestandteile Methylacetat, Methanol, Dimethylether und Methyljodid von den höher siedenden Bestandteilen abzutrennen und die vorhandene Essigsäure mit dem im molaren Überschuss vorliegenden Methanol zu Methylacetat zu verestern. Da das Methylacetat laufend aus dem Gleichgewicht entfernt wird, gelingt die Veresterung zwanglos, sofern man durchschnittliche Verweilzeiten von 5 bis 30 min für das Reaktionsgut in der Kolonne D1 einhält. Die durchschnittlichen Verweilzeiten lassen sich mittels des Rücklaufverhältnisses und/oder mittels der Einstellung der Höhe der Überlaufwehre an Glockenböden oder Böden ähnlicher Konstruktion einstellen. Meistens genügen jedoch einfache Füllkörperkolonnen. Zweckmässigerweise hält man einen Veresterungsgrad von 60–80% ein, weil die restliche Essigsäure den Katalysatorkreislauf erleichtert.

Die Anzahl der Trennstufen in D1 beträgt zweckmässigerweise 5 bis 15 theoretische Böden.

Im Verfahrensschritt (c) wird die Kopffraktion von D1 in der Destillationskolonne D2 in eine mengenmässig geringe Kopffraktion aus Dimethylether, Methyljodid und kleineren Anteilen Methanol und Methylacetat und eine Sumpffraktion aus Methanol und Methylacetat zerlegt. Hierfür verwendet man Kolonnen beliebiger Bauart – aus wirtschaftlichen Gründen vorzugsweise Füllkörperkolonnen – mit etwa 10 bis 20 theoretischen Böden. Die Kopffraktion von D2 wird dann unmittelbar in den Reaktor R zurückgeführt, da sich Dimethylether bei der Carbonylierung wie Methanol verhält.

Die Sumpffraktion von D1 wird gemäss Verfahrensschritt (d) der Kolonne D3 zugeführt, in welcher der Grossteil des Wassers, welches bei der

Carbonylierung und der Veresterung laufend gebildet wird, aus dem Kreislauf entfernt wird. Die Sumpffraktion, welche das restliche Wasser, etwas Essigsäure sowie die Katalysatorkomponenten (den Metallkomplex und gegebenenfalls den überschüssigen Liganden) enthält, wird in den Reaktor R zurückgeführt. Sind auch noch höher siedende Carbonylierungsprodukte vorhanden, so entfernt man diese entweder als Seitenabzug von D3 und/oder in einer kontinuierlich oder diskontinuierlich zusätzlichen Operation, die man zwischen den Sumpf von D3 und R schaltet. Eine derartige Massnahme, die wegen Regenerierung des Katalysators von Zeit zu Zeit erforderlich ist, entspricht der üblichen Technik bei kontinuierlichem Verfahren mit geschlossenen Stoffkreisläufen und bedarf deshalb keiner näheren Erläuterung.

Die Anzahl der theoretischen Böden in der Kolonne D3 beträgt etwa 5 bis 15. Die Bauart der Kolonne D3 ist beliebig, so dass man auch hier zweckmässigerweise eine Füllkörperkolonne verwendet.

Im Verfahrensschritt (e) wird das vornehmlich aus Methylacetat und Methanol bestehende Sumpfprodukt von D2 im Hydrierreaktor H in an sich bekannter Weise zu einem Gemisch aus Methanol und Ethanol hydriert. Vorzugsweise verwendet man hier einen Kupfer/Chromit-Kontakt (sog. Adkins-Katalysatoren) oder einen Kupferoxidkontakt bei einem Waserstoffdruck von 100–300 bar und einer Temperatur von ca. 180–250°C. Diese Hydrierung gelingt glatt, jedoch muss man für einen möglichst vollständigen Umsatz sorgen, damit sie nicht zum Teil auf Zwischenstufen wie Aldehyden, Acetalen, Ethern und Estern stehen bleibt. Die Raum-Zeit-Ausbeute bei der Hydrierung beträgt etwa 0,5–1,0 kg/l/h.

Im Verfahrensschritt (f), der ebenfalls in an sich bekannter Weise vorgenommen wird, wird das in H erhaltene Reaktionsgemisch in seine Komponenten zerlegt. Die hierzu erforderliche Destillationskolonne D4 kann beliebiger Bauart sein (vorzugsweise verwendet man eine Füllkörperkolonne) und soll etwa 15 bis 25 theoretische Böden haben. Höher siedende Produkte, welche im Laufe des Verfahrens gebildet und bis nach D4 mitgeschleppt wurden, werden als Sumpfprodukt entfernt. Als Kopffraktion fällt das Methanol an, welches noch etwas Methylacetat enthalten kann. Dieses Kopfprodukt wird in die Synthesestufe zurückgeführt. Man erhält das Verfahrensprodukt Ethanol, welches man D4 flüssig oder dampfförmig als Seitenabzug entnimmt in technischer Reinheit (99%) sowie in einer Ausbeute von 95 bis 96%, bezogen auf eingesetztes Methanol.

Alle Destillationsschritte können bei Normaldruck oder – aus kühltechnischen Gründen - bei leicht erhöhtem Druck vorgenommen werden.

Das erfindungsgemässe Verfahren liefert nicht nur hohe Ethanol-Ausbeuten, sondern löst das Problem der Aufarbeitung von Carbonylierungsgemischen auf technisch besonders elegante Weise. Hierbei ist speziell hervorzuheben, dass die häufig schwierige Abtrennung, Regenerierung und Rückführung aller katalytisch aktiven Substanzen mittels der erfindungsgemässen Kreisläufe verfahrenstechnisch besonders einfach und wirtschaftlich gelingt.

Beispiel

Die Herstellung des Ethanols erfolgte in einer Versuchsanlage nach Fig. 1, wobei die einzelnen Verfahrensschritte bis auf die Hydrierung zwar diskontinuierlich vorgenommen werden, in ihrer Gesamtheit jedoch einem Modellversuch für die Übertragung in die kontinuierliche Arbeitsweise entsprachen. Die angegebenen Werte entstammen dem 5. Zyklus eines insgesamt 7mal zur Erreichung eines quasi-stationären Ablaufes wiederholten Versuches.

In diesem Ablauf wurden die in den Reaktoren und Kolonnen jeweils zurückbleibenden Stoffmengen nicht mehr berücksichtigt.

a) Carbonylierung in R

Die Carbonylierung wurde in einem Tantal-Schüttelautoklaven R von 1 l Rauminhalt unter einem CO-Druck von 40 bar bei 175°C im Laufe von 1 h in Gegenwart eines Rh/J/Triphenylphosphin-Katalysatorsystems vorgenommen.

Die Einsatzstoffgemische setzten sich wie folgt zusammen:

| | frisch | rück-geführt | gesamt |
|---|---|---|---|
| Methanol | 138 g | 204 g | 342 g |
| Methylacetat | – | 35 g | 35 g |
| Essigsäure | – | 12 g | 12 g |
| Dimethylether | – | 49 g | 49 g |
| Wasser | – | 17 g | 17 g |
| Rh-Katalysator | – | 2 g | 2 g |
| $PPh_3$ (Ph = Phenyl) | – | 2 g | 2 g |
| $CH_3J$ | – | 32 g | 32 g |

Als Rh-Katalysator diente der Komplex $Rh(CO)(PPh_3)_2Cl$. Das Verhältnis Rh zu Methanol betrug $7 \cdot 10^{-3}$ mol/kg, das molare Verhältnis des freien $PPh_3$ zu Rh 4:1 und das molare Verhältnis von J zu Rh 100:1.

Das J wurde frisch als wässrige Jodwasserstoffsäure eingesetzt und in rückgeführter Form lag es praktisch vollständig als Methyljodid vor.

Der Reaktoraustrag setzte sich wie folgt zusammen:

| | |
|---|---|
| Methylacetat | 238 g |
| Essigsäure | 99 g |
| Dimethylether | 51 g |
| Methanol | 123 g |
| Wasser | 70 g |
| Katalysatorbestandteile | 4 g |
| sonstige Produkte ($CH_3J$ u.a.) | 35 g |
| gesamt | 620 g |

Dies entspricht einem Methanolumsatz von 64,0%, wobei die Selektivität bezüglich des Methylacetats rd. 65% und die zu Essigsäure rd. 35% betrug. Rückgeführter und in gleicher Menge gebildeter Dimethylether blieb hierbei ausser Betracht.

b) Destillation in D1

Der Reaktoraustrag wurde in einer 1,8 m hohen Füllkörperkolonne D1, die rd. 20 theoretische Böden hatte, bei Normaldruck in zwei Fraktionen folgender Zusammensetzung zerlegt:

| | |
|---|---|
| Kopffraktion | |
| Methylacetat | 343 g |
| Dimethylether | 49 g |
| Methanol | 77 g |
| Methyljodid | 32 g |
| Wasser | 1 g |
| gesamt | 502 g |

| | |
|---|---|
| Sumpffraktion | |
| Essigsäure | 12 g |
| Wasser | 95 g |
| Katalysatorbestandteile | 4 g |
| sonstige Produkte | 3 g |
| gesamt | 114 g |

Die Rate der Veresterung der Essigsäure mit Methanol zu Methylacetat betrug 87,5%.

c) Destillation in D2

Die Kopffraktion von D1 wurde in einer 1,8 m hohen Füllkörperkolonne D2, die rd. 20 theoretische Böden hatte, bei Normaldruck in die folgenden beiden Fraktionen zerlegt:

| | |
|---|---|
| Kopffraktion | |
| Methylacetat | 22 g |
| Dimethylether | 49 g |
| Methanol | 5 g |
| Methyljodid | 32 g |
| Wasser | 1 g |
| gesamt | 109 g |

| | |
|---|---|
| Sumpffraktion | |
| Methylacetat | 321 g |
| Methanol | 71 g |
| gesamt | 392 g |

d) Destillation in D3

Die Sumpffraktion von D1 wurde in einer 1,2 m hohen Füllkörperkolonne D3 (rd. 10 theoretische Böden) bei Normaldruck in folgende Fraktionen zerlegt:

| | |
|---|---|
| Kopffraktion | |
| Wasser | 79 g |

| | |
|---|---|
| Sumpffraktion | |
| Wasser | 16 g |
| Essigsäure | 12 g |
| Katalysatorbestandteile | 4 g |
| sonstige Produkte | 3 g |
| gesamt | 35 g |

e) Hydrierung in H

Die Hydrierung der Sumpffraktion von D2 in einem 2,0-l-Reaktor wurde bei 190°C und einem Wasserstoffdruck von 280 bar kontinuierlich mit einer Katalysatorbelastung von 500 g Methylacetat pro Stunde und pro Liter Festbettkontakt vorgenommen. Als Kontakt diente ein handelsüblicher Katalysator aus Tabletten von 5 mm Durchmesser und 3 mm Schichtstärke, der in freier Schüttung den Reaktor ausfüllte und folgende Zusammensetzung (Gew.%) hatte: 34,4% $SiO_2$; 36,0% CuO; 17,2% MgO; 1,0% BaO; 1,1% $Cr_2O_3$ und 0,6% ZnO.

Der Reaktoraustrag bestand aus

| | |
|---|---|
| Ethanol | 189 g |
| Methanol | 199 g |
| Methylacetat | 13 g |
| sonstige Produkte | 3 g |
| gesamt | 404 g |

Dies entspricht einer 96,1%igen Hydrierung des Methylacetats, wobei die Selektivität bezüglich des Ethanols 99,0% betrug.

f) Destillation in D4

Der Reaktoraustrag aus dem Hydrierreaktor wurde bei Normaldruck in einer 4 m hohen Glokkenbodenkolonne (rd. 15 theoretische Böden) in folgende Fraktionen zerlegt:

| | |
|---|---|
| Kopffraktion | |
| Methanol | 199 g |
| Methylacetat | 13 g |
| gesamt | 212 g |

Seitenabzug auf Höhe des 3. Bodens von unten

| | |
|---|---|
| Ethanol | 189 g |

| | |
|---|---|
| Sumpffraktion | |
| höher siedende Bestandteile | 0,4 g |

Bezogen auf das in R insgesamt eingesetzte Methanol betrug die Ethanolausbeute somit rd. 40%. Bezogen auf das frisch zugeführte Methanol errechnet sich eine Selektivität bezüglich des Ethanols von 95,5%.

Die Kopffraktion von D2 und D4 sowie die Sumpffraktion von D3 wurden zusammen mit den entsprechenden Mengen frischer Einsatzstoffe wieder in den Reaktor R zurückgeführt und einem weiteren Versuchszyklus unterworfen. Bei mehrfacher Wiederholung dieses Zyklus stellte sich die qualitativ und quantitativ praktisch konstante Mengenbilanz ein, die im Vorstehenden angegeben wurde.

**Patentanspruch**

Verfahren zur kontinuierlichen Herstellung von Ethanol auf dem Wege über die Carbonylierung von Methanol, dadurch gekennzeichnet, dass man

a) Methanol in Gegenwart eines Carbonylkomplexes eines Metalles der VIII. Gruppe des

Periodensystems sowie einer Halogenverbindung als Aktivator in an sich bekannter Weise in einem Reaktor R carbonyliert,

b) den im wesentlichen aus Methylacetat, Methanol, Essigsäure, Wasser sowie geringen Mengen Dimethylether, Methyljodid und, falls kein fest angeordneter Katalysator verwendet wird, dem Katalysator bestehenden Reaktoraustrag in einer Destillationskolonne D1 in eine Kopffraktion aus Methylacetat, Methanol, Dimethylether und einer halogenorganischen Verbindung einerseits und eine Sumpffraktion aus Wasser, geringen Mengen Essigsäure und dem Katalysator andererseits trennt, wobei man die Verweilzeit so einstellt, dass der Grossteil der Essigsäure mit dem vorhandenen Methanol zu Methylacetat reagiert,

c) die Kopffraktion aus D1 in einer Destillationskolonne D2 in eine Kopffraktion aus geringen Mengen Methylacetat, Methanol, Dimethylether und der halogenorganischen Verbindung einerseits und eine Sumpffraktion aus Methylacetat und Methanol andererseits zerlegt und die Kopffraktion nach R zurückführt,

d) aus der Sumpffraktion von D1 in einer Destillationskolonne D3 den Grossteil des Wassers über Kopf abdestilliert und aus dem Kreislauf entfernt und die aus geringen Mengen Wasser, Essigsäure und dem Katalysator bestehende Sumpffraktion nach R zurückführt und

e) die Sumpffraktion von D2 in den Hydrierreaktor H in an sich bekannter Weise mit Wasserstoff zu einem Gemisch aus Methanol und Ethanol hydriert, welches

f) in einer Destillationskolonne D4 in Ethanol und Methanol zerlegt wird, wonach das Methanol nach R zurückgeführt wird.

**Claim**

A process for the continuous production of ethanol via the carbonylation of methanol, wherein

a) methanol is carbonylated in a conventional manner in a reactor R in the presence of a carbonyl complex of a metal of group VIII of the periodic table and of a halogen compound as activator,

b) the reactor discharge, which consists essentially of methyl acetate, methanol, acetic acid, water and small quantities of dimethyl ether, methyl iodide and the catalyst, if the latter is not in a fixed bed, is separated in a distillation column D1 into a top fraction comprising methyl acetate, methanol, dimethyl ether and an organohalogen compound, and into a bottom fraction comprising water, small quantities of acetic acid and the catalyst, the residence time being so adjusted that the bulk of the acetic acid reacts with the methanol present to give methyl acetate,

c) the top fraction from D1 is separated in a distillation column D2 into a top fraction comprising small quantities of methyl acetate, methanol, dimethyl ether and the organohalogen compound, and a bottom fraction comprising methyl acetate and methanol, and the top fraction is recycled to reactor R,

d) the bulk of the water is distilled off, via the top of distillation column D3, from the bottom fraction from D1 and removed from the recycle loop, and the bottom fraction consisting of small quantities of water, acetic acid and the catalyst is recycled to R,

e) the bottom fraction from D2 is hydrogenated with hydrogen in a conventional manner in the hydrogenation reactor H to give a mixture of methanol and ethanol, and

f) the mixture is separated into ethanol and methanol in a distillation column D4, following which the methanol is recycled to R.

**Revendication**

Procédé de préparation continue de l'éthanol par carbonylation du méthanol, caractérisé en ce que:

a) on carbonyle de manière connue en soi, dans un réacteur R, le méthanol en présence d'un complexe carbonylé d'un métal du groupe VIII de la Classification Périodique et d'un composé halogéné qui sert d'activateur,

b) on sépare le produit sortant du réacteur et qui consiste essentiellement en acétate de méthyle, méthanol, acide acétique, eau et petites quantités d'éther diméthylique, d'iodure de méthyle et, si l'on n'a pas utilisé un catalyseur fixe, de catalyseur, dans une colonne à distiller D1, en une fraction de tête consistant en acétate de méthyle, méthanol, éther diméthylique et un composé organique halogéné d'une part, et une fraction de culot consistant en eau, petites quantités d'acide acétique et catalyseur d'autre part, en réglant la durée de passage de manière que la plus grande partie de l'acide acétique réagisse avec le méthanol présent avec formation d'acétate de méthyle,

c) on sépare la fraction de tête provenant de la colonne D1, dans une colonne à distiller D2, en une fraction de tête consistant en petites quantités d'acétate de méthyle, méthanol, éther diméthylique et composé organique halogéné d'une part et une fraction de culot consistant en acétate de méthyle et méthanol d'autre part, et on recycle la fraction de tête au réacteur R,

d) à partir de la fraction de culot de la colonne D1, on distille la plus grande partie de l'eau en tête dans une colonne à distiller D3 et on l'élimine du circuit, et on recycle la fraction de culot consistant en petites quantités d'eau, acide acétique et catalyseur, au réacteur R, et

e) on hydrogène la fraction de culot de la colonne D2 dans le réacteur d'hydrogénation H, de manière connue en soi, par l'hydrogène, avec formation d'un mélange de méthanol et d'éthanol, lequel

f) est séparé dans une colonne à distiller D4 en éthanol et méthanol, ce dernier étant recyclé au réacteur R.

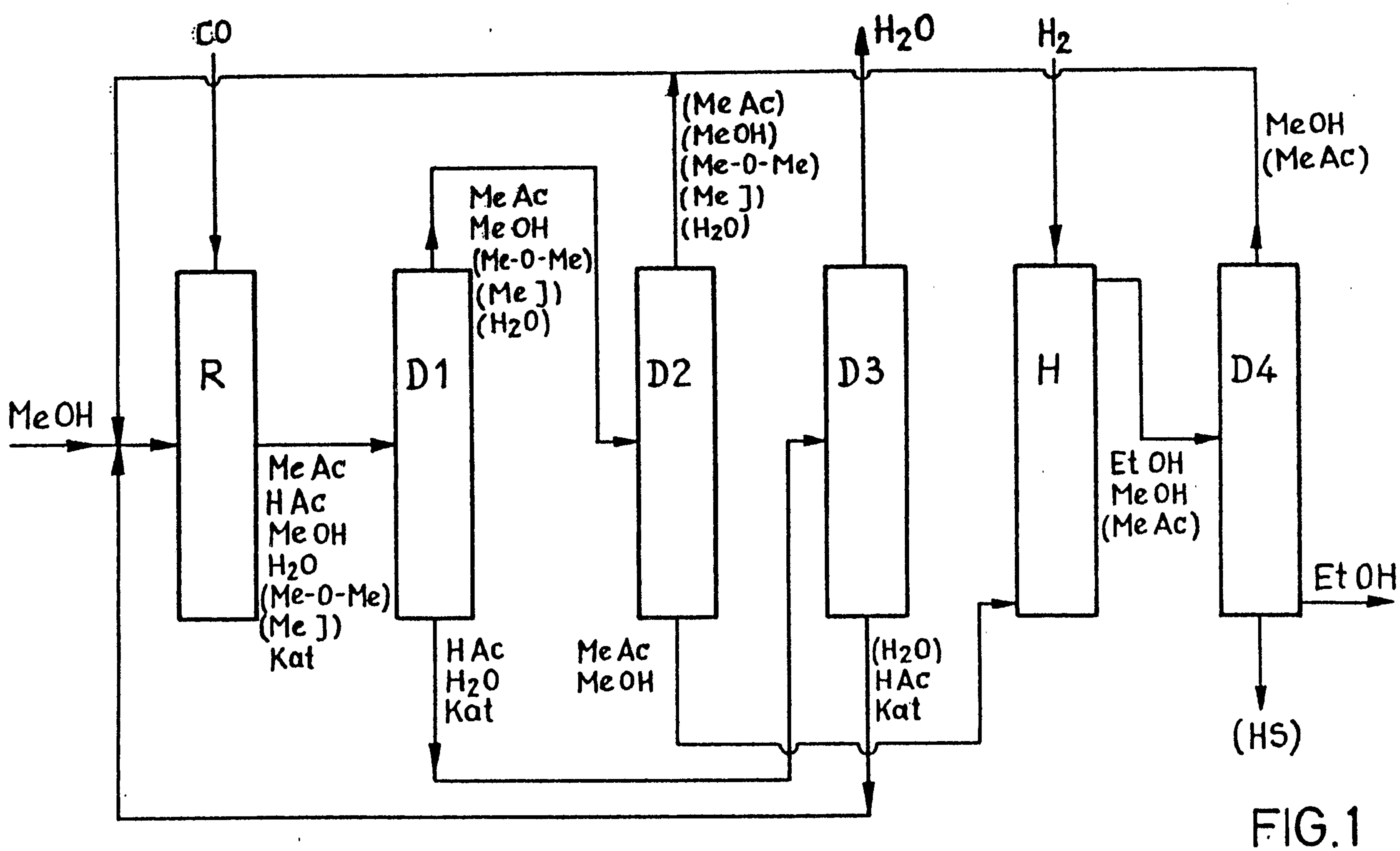


FIG.1

HAc = Essigsäure
MeOH = Methanol
MeAc = Methylacetat

Me-O-Me = Dimethylether
MeJ = Methyljodid
Kat = Katalysator

EtOH = Ethanol
HS = Höher siedende Nebenprodukte
( ) = geringe Mengen

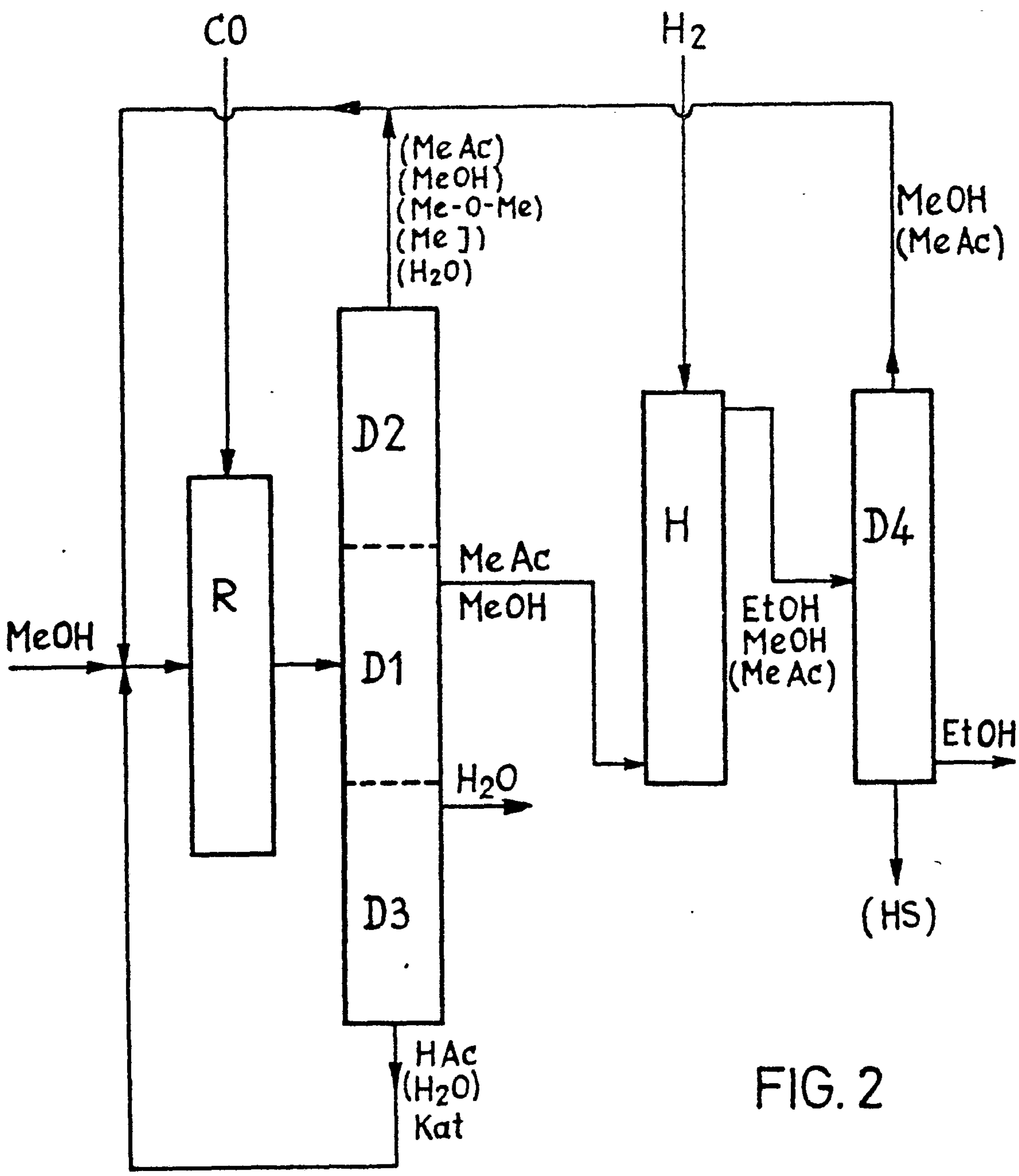


FIG. 2

Legende s.Fig.1